# EUROPEAN PATENT APPLICATION

(11) **EP 0 692 264 A2**
(43) Date of publication of application: **17.01.1996**
(21) Application number: 95304206.6
(22) Date of filing: 16.06.1995
(51) Int. Cl.: A61L 27/00, C08L 27/12, C08L 27/16, B29C 47/00

(54) **Expanded PTFE implantable prosthesis with improved blood and tissue compatibility and superior patency**

(30) Priority: 11.07.1994 US 273326
(71) Applicant: Meadox Medicals, Inc., Oakland New Jersey 07436 (US)
(72) Inventor: Zdrahala, Richard J., Montville, NJ 07045 (US); Lentz, David J., Randolph, NJ 07869 (US); Patnaik, Birendra K., Chester, NJ 07930 (US)
(74) Representative: Jones, Michael Raymond

(57) **Abstract**

Synthetic composite materials for surgical implantation are disclosed which are manufactured from a fluoropolymer and a copolymer of a perfluorosulfonate, the other comonomer optionally being a perfluorinated monomer. The materials may be manufactured by coextrusion of the fluoropolyumer and the perfluorosulfonate copolymer. Alternatively, the perfluorsulfonate copolymer may be attached to the surface of an extruded fluoropolymer material. Preferably, the fluoropolymer is polytetrafluoroethylene and the perfluorosulfonate copolymer is a copolymer of perfluoro-3,6-dioxa-4-methyl-7-octane sulfonyl fluoride and tetrafluoroethylene. In a prepared embodiment a synthetic vascular graft is prepared from extruded and expanded polytetrafluoroethylene, coated with a perfluorosulfonate copolymer optionally converted to an alkali salt form and subsequently heated to a temperature above the crystalline melting point of polytetrafluoroethylene.

## Description

### BACKGROUND OF THE INVENTION

### 1. Field of the Invention

The present invention relates generally to synthetic composite materials for surgical implantation and to the methods of making such materials. In a specific aspect, the present invention relates to implantable materials suitable for use as vascular prostheses, exhibiting improved properties of antithrombogenicity, as well as improved cellular adherence and growth.

### 2. Description of the Related Art

An ideal implantable prosthesis will closely approximate the physical and physiological characteristics of normal body tissue. As a result, a variety of luminal prosthetic materials and structures have been manufactured in attempts to produce just such a prosthesis. While significant progress has been made along many of the parameters defining ideal prostheses, no material or structure has heretofore produced truly optimal performance.

An implantable tubular prosthesis should be biocompatible, resisting degradation or encapsulation by adjacent tissues, and causing neither mutagenic nor allergic responses in the organism. In addition, the prosthesis must be flexible and strong, as well as resistant to tearing, puncture, aneurism and collapse. Among the luminal devices used as conduits for repairing or replacing natural tubular structures are those which serve as conduits for blood, such as endovascular prostheses and vascular grafts. These luminal devices, besides requiring the above-mentioned properties, must also avoid inciting excessive thrombotic responses in the blood they convey.

It has been found that prostheses which effectively avoid the formation of thrombus develop endothelial linings or neointima. The neointima arise through the deposition of adhesion glycoproteins on the interior surface followed by fibrin deposition and endothelial cell migration and growth. The neointimal lining is desirably limited in depth, but commonly exceeds normal epithelial growth limitations (e.g., depth of fibrin layer, confluence of sheet) to, in the case of smaller prostheses, constrict or eliminate the lumen of the synthetic portion of the blood vessel.

Among the measures observed to reduce such vascular compromise, the introduction of pores into the walls of the prosthesis has proven effective. In larger diameter prostheses, such pores are often a product of the woven material used as the prosthetic structure. In smaller vessels, woven materials have been found to be less desirable, and preferred materials include porous fluoropolymers. The porosity of these materials has been found to be a critical factor in their utility as implantable prostheses, since ingrowth of tissue onto the walls of the prostheses directly improves their biocompatibility.

Most vascular prostheses are used to replace larger blood vessels, e.g., over 6 mm internal diameter, such as the aorta and other major arteries. Currently available replacements for these larger vessels generally successful, resisting complete occlusion by the overgrowing neointima, but commonly suffering from an unacceptable degree of narrowing. Prostheses having smaller internal diameters, e.g., less than 6 mm, vascular prostheses, such as those used for replacement and repair of coronary arteries and peripheral vessels, suffer more severely from low patency rates, tending to become more or less completely occluded and, as a result, useless for their intended purpose.

Recent efforts to develop suitable small diameter vascular prostheses have been hampered by an inability to find a material which promotes the development of a healthy neointimal lining. If a proper surface is provided, such a lining is formed by the adsorption of proteins in the blood onto the surface followed by platelets and leukocyte adherence and fibrin polymerization, resulting in growth of a surface layer which includes a layer of endothelial cells in direct contact with the blood. If the luminal lining overdevelops, thrombi (bloodclots) can occur. Another type of problem arises if the lining does not adhere well to the inner surface of the prosthesis. In this case, embolization can occur, when all or part of the neointima detaches and can become trapped in small blood vessels. The surface morphology, or surface topography, of the implant has been shown to have a major effect on the adherence and development of the neointima lining.

The term "biocompatible" as employed herein describes a material that is relatively non-thrombogenic when used in direct contact with blood and that is generally compatible with tissue. Biocompatibility therefore entails that the material be hypoallergenic, preferably non-allergenic, as well as non-mutagenic and non-carcinogenic over the long term when implanted.

Various theories concerning the basis for biocompatibility of polymeric materials and devices have been advanced over recent years. While biocompatibility is still not completely understood, it is generally agreed that a limited set of parameters determines whether a material is safely implantable into living organisms. These parameters generally can be divided into two categories: (1) structural parameters and (2) materials parameters.

Structural parameters that bear on a material's biocompatibility principally include the mechanical properties, porosity, and microscopic surface features of the material. In a vascular prosthesis, the physiological compliance of the prosthesis is dependent upon matching the mechanical properties, e.g., resistance to deformation, of the host vessel and prosthetic material. On the other hand, the selection of the level of porosity and surface structure of the material is concerned more with those properties that will permit the neointimal tissue to grow into the prosthesis, anchoring the neointimal lining and promoting long-term survival.

Materials parameters that influence a material's biocompatibility include the hydrophilic/hydrophobic balance, surface energy, and chemical and electrical nature of the material's surfaces. Thus, it has been proposed that the higher the water content of a material, a factor determined by the material's charged or polar constituents, the more closely it will mimic the properties of natural tissue and the greater the degree of biocompatibility. Accordingly, hydrophilic materials have generally been found to be more desirable for implantation. Similarly, it has been proposed that if the surface energy of a synthetic material matches that of natural tissue, enhanced biocompatibility will result.

The thickness of the fibrin layer varies, for example, according to the prosthesis material and its surface structure. Since the thickness of the fibrin layer approaches 0.5 to 1 mm when a knitted or woven fabric of Dacron or polytetrafluoroethylene (hereinafter also referred to as PTFE) is used as the prosthesis, success is achieved only with those prostheses which do not become unacceptably narrowed or occluded due to this increase in wall thickness. In effect, only larger prostheses, i.e., those having an inside diameter of 5 to 6 mm or more, may be usefully fabricated from woven or knitted fabric materials. Generally, knitted or woven prostheses having smaller inner diameters have not been successful.

A fluoropolymer tubing which has been stretched has, on the other hand, a microstructure composed of very fine fibers, i.e., microfibrils, and nodes connected together by the fibrils. The diameters of the fibrils are dependent on a variety of conditions, and can be made much smaller than fibers of the knitted and woven fabrics mentioned above.

Platelets adhere to the adsorbed plasma protein to form fibrin fibers which capture blood corpuscles and become a fibrin deposited layer. This deposited layer is expected to subsequently form a pseudointima of the prosthesis. However, the fibrin layer frequently over-develops, becoming too thick. As a result, insufficient nutrition of the pseudointima or neointima occurs. This in turn will result in tissue necrosis and, commonly, a delamination of the pseudointima from the prosthesis wall or thrombic occlusion of the inner surface of the prosthesis.

The design and selection of materials useful in vascular prostheses therefore requires an understanding of the physical and chemical characteristics of the materials necessary for irreversible endothelialisation of a surface and for inhibition of undesirable platelet interactions.

The mechanism by which cells adhere to polymer surfaces is only partially understood, but the initial processes of cellular attachment are thought to be promoted by adhesive glycoproteins such as fibronectin and vitronectin adsorbed from the serum component of blood onto the polymeric surface. Several lines of evidence suggest that for biomaterials where avid cell attachment is required, the use of hydrophilic polymers may be preferable. A number of studies have shown that where the surfaces are not deliberately precoated with purified Fn, hydrophilic surfaces are generally superior to hydrophobic polymers for attachment of cells including human endothelial cells. Precoating with a high concentration of Fn enables initial attachment of fibroblasts or endothelial cells on to a number of hydrophobic surfaces to be achieved, but studies with the polystyrene hydrophobic/hydrophilic model system showed that the molar potency of the Fn adsorbed on to hydrophilic "tissue culture" polystyrene was six-fold higher than Fn adsorbed on to non-wettable polystyrene. Another consideration is that the maintenance of a normal endothelial cell phenotype in vivo is likely to be dependent in part upon the ability of the cells to secrete and assemble a "normal" extracellular matrix. In view of the hydrophilic nature of extracellular matrix components, a hydrophilic polymer surface might be expected to be superior to a non-wettable surface for extracellular matrix assembly and adhesion.

One of the notable features of fluoropolymers, and PTFE in particular, is the extraordinarily low surface energy of articles made therefrom. This low surface energy is manifested, for example, by the extraordinary resistance of fluoropolymers to chemical modification, e.g., high resistance to strong acids and bases. The low surface energy of such fluoropolymers also manifests in a very high degree of hydrophobicity or resistance to wetting. A consequence of the low wettability of fluoropolymeric materials is that cells, especially epithelial cells such as endothelial cells, tend not to adhere to the surfaces of the materials. This lack of adherence inhibits the formation of epithelial sheets and reduces the structural integrity of any sheets that do form. A factor critical for the formation of neointima on the interior of vascular prostheses, then, is a reduction of the hydrophobicity of the prosthesis material.

The failure of hydrophobic polymers such as fluoropolymers to adequately support cell attachment, including attachment of endothelial cells exposed to the shear forces inherent in blood flow, is a limitation to the use of such materials for vascular prostheses. If a fluoropolymer could be chemically modified to produce a surface that supported enhanced endothelial cell attachment and growth, then the modified surface could be expected to be more suitable than an unmodified fluoropolymer for supporting the process of in vivo endothelialisation. A modified fluoropolymer exhibiting superior qualities for the attachment of endothelial cells and neointimal development would certainly be preferable for use in procedures employing the pre-seeding of grafts with endothelial cells prior to implantation.

Historically, attempts to improve the chemical parameters of surfaces of biomaterials have focused on modifying the surfaces to increase their hydrophilicity or wettability. Methods of modifying the chemical characteristics of polymer surfaces generally fall into either of two categories. One type of approach involves modifying the materials or compounds used to manufacture articles for implantation. An example of this approach includes forming alloys of a polymeric precursor with compounds capable of modifying the physical and chemical properties of the resulting polymer. Another approach to modifying polymer surfaces involves manufacturing the polymeric material or article and subsequently altering its surface. Examples of this approach include applying coatings to the articles or modifying the articles chemically.

The ability to form alloys of various fluoropolymers is well known in the art. U.S. Patent No. 4,973,609, for example, describes the use of alloys of a variety of fluoropolymers, each individually possessing distinct physical characteristics, to yield materials possessing physical characteristics different from materials produced from single fluoropolymers. The alloy materials disclosed in this patent are described as capable of extrusion into tubular form.

Alloys of fluoropolymers with other materials are also known. For example, U.S. Patent Nos. 4,219,520 and 4,254,180 describe methods for producing extrudable alloys of fluoropolymers and a particulate such as graphite or silica. These patents also describe coating methods to improve the wettability and anti-thrombogenicity of the materials. U.S. Patent No. 5,141,522 describes three-part alloy composites made from PTFE, a thermoplastic polymer, and a bioabsorbable material. The bioabsorbable material is described as a polymer of monomers such as lactides and carbonates. The benefit of these alloys is alleged to be improved tissue ingrowth into the interior of the composite when implanted in mammals.

Another alloy material is described in U.S. Patent No. 4,822,615. In this patent, the alloy material is described as being made from an extrudable mixture of a hydrophobic resin, a hydrophilic resin, and a solution of heparin in glycerol. Heparin is a well-known anticoagulant, and the heparin alloy is described as possessing improved anti-thrombic properties. This patent does not, however, suggest the use of a fluoropolymer as an alloy component, relying instead on other types of polymers.

As was mentioned above, the chemical properties of polymeric surfaces can also be modified after the manufacture of articles destined for implantation. For example, a variety of methods of applying coatings to synthetic biomaterials are known in the art. Such methods are described, for example, in U.S. Patent Nos. 4,254,180, 4,219,520 and 4,349,467 each describe methods for applying coatings of heparin to reduce the hydrophobicity and thrombogenicity of articles formed from PTFE. U.S. Patent No. 4,113,912 describes forming porous fluorocarbon articles, the pores of which have been coated with a water-insolubilized water soluble polymer, such as an oxygen-containing hydrocarbon polymer. The hydrophilic coating is cross-linked to the fluorocarbon article to prevent loss of the coating in aqueous media.

U.S. Patent No. 5,118,524 describes a method for making a vascular biomaterial, the method including chemically modifying a PTFE article and then covalently bonding to the article a hemocompatible membrane. The hemocompatible membrane is described as a layer of phospholipids.

It has been observed that the antithrombogenicity of a material is linked to the polarity or charge of substituents present on or pendent from the surface of the material. Therefore, attempts have been made to modify such substituents or add polar and/or charged substituents to the surfaces of proposed biomaterials. In particular, sulfonation of polymeric surfaces has been attempted. U.S. Patent No. 5,116,361, for example, describes the formation of articles from polymers of sulfonated polyethylene oxide. U.S. Patent No. 5,010,009 describes articles made from fluoropolymers, to which have been grafted polyacrylic acid chains. The acrylic moieties are subsequently modified with sulfuric acid to produce sulfonate groups.

An exemplary fluorocarbon polymer having pendent sulfonic groups is the chemically inert, non-crosslinked cation-exchange resin known by the trademark NAFION®. NAFION® is chemically identified as a copolymer of tetrafluoroethylene and perfluoro-3,6-dioxa-4-methyl-7-octene sulfonyl fluoride. The mechanical and chemical stability of this perfluorosulfonate ionomer and its selective permeability to charged ions have made it useful for, among other things, industrial electrochemical separating processes. Moreover, this perfluorosulfonate (hereinafter also referred to as PFS) copolymer is also hydrophilic, in contrast to PTFE and other fluoropolymers which are substantially hydrophobic. NAFION® and its related PFS copolymers are also easily manipulated, allowing the formation of films and castings.

Several patents describe methods of employing a fluorocarbon copolymer as a coating for articles made from PTFE. For example, U.S. Patent Nos. 4,778,723 and 4,698,243 describe methods for coating PTFE fibers and yarns to modulate the size of the fibers. The coatings comprise a perfluorinated polymer and a fluorinated monomeric treating agent. The fluorinated polymer may be selected from a variety of polymers including copolymers of a fluorinated monomer and perfluoro-3,6-dioxa-4-methyl-7-octene sulfonyl fluoride. These coated articles are not described as useful for vascular prostheses, nor are the coatings described in relation to cellular growth or attachment.

U.S. Patent No. 5,077,215 describes coatings for biocompatible articles. The articles may be manufactured from any of various polymers, preferably PTFE. The coatings comprise a copolymer of a monomer, preferably TFE, and perfluoro-3,6-dioxa-4-methyl-7-octene sulfonyl fluoride. The most preferred coating is the copolymer of TFE and perfluoro-3,6-dioxa-4-methyl-7-octene sulfonyl fluoride marketed as NAFION®. These products are described as exhibiting reduced thrombogenicity and enhanced attachment and growth of adherent animal cells. While the patent mentions the possibility of casting the perfluorosulfonate copolymer to form a vascular prosthesis, it does not describe or suggest processes for the extrusion of the copolymer. Nor does this patent describe or suggest the co-extrusion of PTFE with a perfluorosulfonate copolymer. Moreover, the patent provides no indication of the advantages of coating an extruded prosthesis with a perfluorosulfonate copolymer.

It is therefore clear that none of the aforementioned patents and publications disclose fluoropolymer materials providing excellent resistance to thrombosis, superior cellular attachment properties, and capable of being extruded by conventional practices to form implantable articles. Other measures have generally been required such as coating a pre-extruded article with an antithrombogenic material, or increasing the complexity of the extrusion method itself.

Therefore, it would be a significant advance in the art to overcome the above-described disadvantages, deficiencies and difficulties associated with producing biocompatible materials for implantation.

According to one aspect of the present invention, there is provided a process for producing a biocompatible fluoropolymer material, comprising the steps of:
(a) forming an extrusion feedstream comprising (i) a first fluoropolymer component comprising a perfluorosulfonate copolymer, and (ii) a second fluoropolymer component comprising a fluoropolymer; and
(b) extruding said extrusion feedstream to form an extrudate.

The second fluoropolymer component may be the same as, but is preferably different from, the first fluoropolymer component.

The first fluoropolymer component may comprise the perfluorosulfonate copolymer and a fluoropolymer (which may be the same as or different from the fluoropolymer of the second fluoropolymer component); and the perfluorosulfonate copolymer may be a copolymer of perfluoro-3,6-dioxa-4-methyl-7-octene sulfonyl fluoride and a tetrafluoroethylene monomer.

The second fluoropolymer component may comprise a fluoropolymer selected from polytetrafluoroethylene, chlorotrifluoroethylene polymers, fluorinated ethylene-propylene polymers, polyvinylidene fluoride, hexafluoropropylene polymers, perfluoroalkoxytetrafluoroethylene polymers, and mixtures thereof, but the fluoropolymer is preferably a polytetrafluoroethylene.

The forming step may comprise combining the first and second fluoropolymer components to form a substantially homogeneous extrusion feedstream.

Alternatively, the forming step may comprise co-feeding the first and second fluoropolymer components; and the extruding step may comprise co-extruding the first and second fluoropolymer components without substantially commingling the fluoropolymer components.

The extruding step may comprise extruding the feedstream to form a tubular extrudate.

The process of the present invention may also include the step of stretching the extrudate at a temperature below the crystalline melting point of the extrudate. Such a process may include sintering the stretched extrudate by heating the extrudate to a temperature above the crystalline melting point of the extrudate.

The extrusion feedstream may include a lubricant. The lubricant may be selected from liquid aliphatic hydrocarbons, aromatic hydrocarbons, alcohols, ketones, esters, silicone oils, fluorocarbon oils, aqueous systems containing surfactants, and mixtures thereof.

One particular process for producing a biocompatible fluoropolymer material, can comprise the steps of:
(a) extruding a fluoropolymer feedstock to form an extrudate;
(b) contacting the extrudate with a treating composition comprising a perfluorosulfonate copolymer and a fluid carrier; and
(c) removing the carrier from the treating composition, thereby depositing the perfluorosulfonate copolymer onto the surface of the extrudate.

In such a process, the perfluorosulfonate copolymer can be a copolymer of perfluoro-3,6-dioxa-4-methyl-7-octene sulfonyl fluoride and a tetrafluoroethylene monomer. The perfluorosulfonate copolymer may be provided as a sodium salt.

In this process the fluid carrier may be water, aqueous solutions of inorganic salts, aqueous solutions of polar organic compounds, polar organic solvents, liquid perhalogenated alkanes, and mixtures thereof.

This particular process may optionally involve stretching the extrudate at a temperature below the crystalline melting point of the extrudate, and/or sintering the stretched extrudate by heating the extrudate to a temperature above the crystalline melting point of the extrudate.

Another aspect of the present invention provides a biocompatible material comprising:
(a) a first fluoropolymer component comprising a perfluorosulfonate copolymer; and
(b) a second fluoropolymer component comprising a fluoropolymer; the

first and second fluoropolymer components having been extruded to form an extrudate.

The different options, and preferred components, as mentioned above in relation to the process aspect of the present invention, are applicable to the biocompatible material of the present invention.

A further aspect of the present invention provides a vascular prosthesis comprising a biocompatible fluoropolymer alloy material, the alloy material comprising a blend of a first fluoropolymer including a copolymer of perfluoro-3,6-dioxa-4-methyl-7-octene sulfonyl fluoride and a tetrafluoroethylene monomer, and a second fluoropolymer including polytetrafluoroethylene; the alloy material having been extruded to form a tubular extrudate.

In one embodiment the feedstream preferably includes at least one fluoropolymer and a perfluorosulfonate copolymer. The feedstream may include a substantially homogeneous mixture of the fluoropolymer and the prefluorosulfonate copolymer. In this embodiment, the extrudate will exhibit a substantially homogeneous or isotropic structure. Alternatively, the feedstream may be asymmetrical, composed of a first feedstock, including the perfluorosulfonate co-polymer, and a second feedstock, including at least one fluoropolymer. In this embodiment, the extrudate will be cross-sectionally inhomogeneous, exhibiting a region substantially composed of a perfluorosulfonate copolymer. The extrudate in this embodiment will exhibit longitudinal homogeneity (i.e., in the dimension along the axis of extrusion), insofar as the two feedstocks are continuously coextruded.

In the embodiment which includes the steps of contacting the extruded fluoropolymer with a solution of a perfluorosulfonate copolymer in a treating agent, and removing the treating agent thereby depositing the perfluorosulfonate copolymer on the surface fluoropolymer extrudate, the extrudate is preferably expanded and sintered prior to being contacted with the perfluorosulfonate copolymer solution.

In yet another embodiment, the invention includes biocompatible materials manufactured from an extruded fluoropolymer and coated with a perfluorosulfonate copolymer. The extruded materials may be stretched, preferably at a temperature below the crystalline melting point of the constituent fluoropolymer. The extrudates may also be sintered by being heated to a temperature above the crystalline melting point of the fluoropolymer. Preferably the perfluorosulfonate copolymer is applied after any expanding step, but before any sintering step. It is preferred that the perfluorosulfonate copolymer be neutralized and converted to the sodium form.

In yet another aspect, the invention relates to improved vascular prostheses having superior biocompatibility and excellent resistance to thrombosis. The prostheses are formed by coextruding a mixture of a fluoropolymer, preferably polytetrafluoroethylene, and a perfluorosulfonate copolymer. Alternatively, the vascular prostheses may be formed by extruding a fluoropolymer and coating the extrudate with a perfluorosulfonate copolymer. The properties of the vascular prostheses of the invention may be improved by stretching the extruded articles, preferably at a temperature below the crystalline melting point of the component polymers. The properties of the prostheses may also be further improved by sintering the extruded and stretched articles by heating the articles to a temperature above the crystalline melting point of the component fluoropolymers.

The preferred perfluorosulfonate copolymer of the invention is a copolymer of perfluoro-3,6-dioxa-4-methyl-7-octene sulfonyl fluoride and a perfluoromonomer, preferably tetrafluoroethylene. It has been observed that the perfluorosulfonate copolymer should be converted to the form of a sodium salt in order to enable the copolymer to survive the elevated temperatures involved in sintering processes.

### DETAILED DESCRIPTION

In one aspect, the present invention relates to a method of making implantable materials exhibiting improved biocompatibility. The method includes the steps of: providing a fluoropolymer feedstream, including at least one fluoropolymer and a perfluorosulfonate copolymer; and extruding the feedstream to yield an improved biocompatible, implantable material.

The method may further include a step of stretching the extruded material, preferably at a temperature below the crystalline melting point of the extrudate. The method may also include a step of sintering the extruded material by heating the material to a temperature above the crystalline melting point of the material.

The feedstream preferably includes at least one fluoropolymer and a perfluorosulfonate copolymer. The feedstream may include a substantially homogeneous mixture of the fluoropolymer and the perfluorosulfonate copolymer. In this embodiment, the extrudate will exhibit a substantially homogeneous or isotropic structure. Alternatively, the feedstream may be asymmetrical, composed of a first feedstock, including the perfluorosulfonate polymer, and a second feedstock, including at least one fluoropolymer. In this embodiment, the extrudate will be cross-sectionally inhomogeneous, exhibiting a region substantially composed of a fluoropolymer and a region substantially composed of a perfluorosulfonate copolymer. The extrudate in this embodiment will exhibit longitudinal homogeneity (i.e. in the dimension along the axis of extrusion), insofar as the two feedstocks are continuously coextruded.

In an alternative embodiment, the feedstream may include a fluoropolymer, and the method then further includes the steps of contacting the extruded fluoropolymer with a solution of a perfluorosulfonate copolymer in a treating agent, and removing the treating agent thereby depositing the perfluorosulfonate copolymer on the surface fluoropolymer extrudate. In this embodiment, the extrudate is preferably expanded prior to being contacted with the perfluorosulfonate copolymer solution. Moreover, in this embodiment the extrudate is sintered after being coated with the perfluorosulfonate copolymer.

In another aspect, the invention relates to biocompatible fluoropolymeric alloy materials comprising a fluoropolymer, preferably polytetrafluoroethylene, and a perfluorosulfonate copolymer that have been mixed and coextruded. The extruded materials may also be stretched, preferably at a temperature below the crystalline melting point of the polymers. The extrudate may also be sintered by being heated to a temperature above the crystalline melting point of the polymers.

In yet another aspect, the invention relates to improved vascular prostheses having superior biocompatibility and excellent resistance to thrombosis. The prostheses are formed by coextruding a mixture of a fluoropolymer, preferably polytetrafluoroethylene, and a perfluorosulfonate copolymer. The properties of the vascular prostheses of the invention may be improved by stretching the extruded articles, preferably at a temperature below the crystalline melting point of the component polymers. The properties of the prostheses may also be further improved by sintering the extruded and stretched articles by heating the articles to a temperature above the crystalline melting point of the component fluoropolymers.

Although vascular and endovascular prostheses and grafts are among the most notable applications of the present invention, prostheses for use in other luminal body structures such as esophageal, intestinal, laryngeal, urethral, ureteral, biliary, various glandular ducts and conduits, and the like are contemplated. These are generally referred to herein collectively as "soft tissue" prostheses.

Among the vascular prostheses that are specifically contemplated are arterio-venous (A-V) shunt grafts, such as those used for dialysis applications, small diameter (3-10 mm) peripheral grafts, tapered grafts, aortic arch grafts, dilatable pediatric grafts, stents, and vein grafts.

Fluoropolymers are characterized by the fact that they are highly inert, paraffinic thermoplastic polymers that have all or some of the hydrogen replaced with fluorine. A variety of fluoropolymer compositions are useful according to the invention, including single and alloyed fluoropolymers manufactured according to processes known in the art. Such fluoropolymers include polymers of tetrafluoroethylene (PTFE), fluorinated ethylene-propylene copolymers (FEP), tetrafluoroethylene copolymers with ethylene, perfluoroalkoxytetrafluoroethylene (PFA), tetrafluoroethylene copolymers with perfluorovinyl ethers, and mixtures thereof. All of these are capable of being extruded, stretched and sintered. Much of the work on development of porous fluoroethylene polymers, and processes for producing porous tetrafluoroethylene polymer materials, have been disclosed in many U.S. patents.

The preferred fluoropolymer is PTFE. In general, any coagulated dispersion PTFE resin may be employed. Preferred PTFE resins include CD 123 and CD 509, available from Imperial Chemical Industries, Bayonne, New Jersey. Such PTFE resins generally are available as powders, having a median particle size in the range of about 500 µm, and an apparent density in the range of about 500 g/L.

Furthermore, the primary requisite of a suitable tetrafluoroethylene polymer resin for the process described above is a very high degree of crystallinity, preferably in the range of 95% or above, and correspondingly low amorphous content. Copolymers of tetrafluoroethylene which have defects in the crystalline structure that introduce a higher amorphous content are less preferred than are homopolymers.

A general procedure for producing porous fluoropolymer products by extrusion may be employed as is described in the art.

Initially, a dispersion of a highly crystalline tetrafluoroethylene polymer powder or coagulated dispersion is mixed with a liquid lubricant and shaped. The lubricant should be capable of wetting the fluoropolymer surface, and of being removed by evaporation or extraction at a temperature below the crystalline melting point of the fluoropolymer. Examples of suitable lubricants include liquid hydrocarbons such as solvent naphtha, white oil, etc.; aromatic hydrocarbons such as toluene, xylene, etc.; alcohols; ketones; esters; silicone oils; fluorocarbon oils; aqueous systems containing surfactants; and mixtures thereof.

A particularly preferred lubricant is a mixed paraffinic hydrocarbon composition available as ISOPAR® from Exxon Chemical Americas, Houston, Texas. ISOPAR® has a boiling point of about 154-176°C.

The amount of lubricant to be used will vary according to the conditions of extrusion, the size of the desired product, and the nature and amount of the fluoropolymers and any additives included in the feedstock. The lubricant may be included in the feedstock in an amount of from about 10 wt.% to about 30 wt.%. Preferably, the lubricant is included in an amount of from about 15 wt.% to about 20 wt.%. Most preferably, the lubricant is included in the feedstock in an amount of about 17.5 wt.%.

The lubricant is then removed from the extrudate. The resulting dried extrudate may then be stretched at a high rate, usually at an elevated temperature which is nonetheless below the crystalline melting point of the tetrafluoroethylene polymer resin. While being held in the stretched state, the tetrafluoroethylene extrudate may be sintered by heating the stretched extrudate to a temperature above the crystalline melting point of the fluoropolymer. This process produces a material having a microstructure composed of nodes interconnected by very small fibers, also known as fibrils or microfibrils. This microstructure greatly increases the tensile strength of the tetrafluoroethylene polymer extrudate. Because of the node and fibril structure, the material is also substantially more porous than the original extrudate.

After extrusion, the fluoropolymer tube may be stretched or "expanded". Expansion is a term well known in the art and may be performed according to the methods known in the art. Generally, expansion involves stretching the extrudate in either the axial or the radial dimension, preferably involving simultaneous stretching in both the axial and radial dimensions. The expanding may be performed at temperatures ranging from about ambient temperature to an elevated temperature that is below the crystalline melting point of the fluoropolymer. The preferred temperature at which the expanding process may be performed is from about 100°C to about 300°C, taking advantage of the fluoropolymer's thermoplastic properties. Preferably, the expanding is performed at a temperature of the extrudate of between about 150°C and about 280°C. Most preferably, the temperature of the extrudate during the expanding step is between about 260°C and about 270°C. The stretching ratio is commonly between about 20% and about 1200%. Preferably, the stretching ratio is between about 200% and about 1000%.

The expansion step is related to modification of several parameters governing the extruded fluoropolymer's behavior. At a gross level, the expansion directly increases the extrudate's diameter and length. Concomitantly, the thickness of the extrudate, i.e., the thickness of the wall of the extruded tube, is decreased. At a microscopic level the microstructure of the extrudate is also affected by the process of expansion. Expansion is well known in the art as a means of altering the size and number of pores in the extrudate. Given the application of the tubes described herein as vascular prostheses, the porosity of the tubes is preferably adjusted by expansion to improve the tube's behavior as functional replacements for vascular tissue. The pores are desirably of a size and number to optimize the endothelial ingrowth accompanying the formation of neointima upon implantation as a vascular replacement.

The temperature and particularly the rate of stretching greatly affect the porosity and tensile strength of the resulting material. Stretching performed at very high rates produces an increase in the strength of the resulting material. When an unsintered extrudate is stretched at a lower rate, limited stretching occurs before fractures occur in the material. Furthermore, materials produced through stretching at lower rates tend to have coarse microstructures and are mechanically weak. Extrudates expanded at both high temperatures and high stretch rates have a more homogeneous structure and a greater tensile strength than extrudates expanded at lower temperatures and lower stretch rates. Therefore, high stretch rates are believed to be necessary to produce strong materials, and both high stretch rates and high temperatures have been recommended to achieve high stretch ratios, homogeneous structures and strong materials.

Furthermore, the primary requisite of a suitable tetrafluoroethylene polymer resin for the process described above is a very high degree of crystallinity, preferably in the range of 98% or above, with a correspondingly low amorphous content. Copolymers of tetrafluoroethylene which have defects in the crystalline structure that introduce a higher amorphous content do not work well in the process as homopolymers.

The process discussed above does not generally produce PTFE materials having fine pores less than 2,000 Å in diameter. The process, however, can be modified to produce a PTFE material having such fine pores by first stretching the extrudate as discussed above, by then "free" sintering the extrudate by heating it above its crystalline melting point without subjecting the extrudate to tension by holding it in its stretched state, and by then stretching the extrudate a second time at a temperature below the crystalline melting point. The second stretching produces a PTFE material having uniform fine pores of between about 100 to about 1500 Å in diameter. Such a process is described, for example, in U.S. Patent No. 4,110,932.

PTFE resin tubes having small pore size but high porosity can be produced by drawing a tubular PTFE extrudate in the lengthwise direction through a metal die and plug to perform the stretching operation. The thickness of the tube can be reduced to a level not previously possible by radially expanding the tube while simultaneously performing the sintering operation.

It has been confirmed clinically that when a structure composed of fibers and nodes is expressed in terms of pore sizes and porosities, or fiber lengths and nodular sizes, a polytetrafluoroethylene tubing, desirably has a pore size of from about 2 µm to about 30 µm. Pore sizes outside this range have been found to be less desirable. The porosity of tubing may be between about 50% and about 90%, expressed as a ratio of the density of the fluoropolymer product to the density of the untreated, source fluoropolymer. Preferably, the porosity of the tubing is between about 70% and about 85%. The fiber length of the extrudate is desirably between about 20 µm and about 110 µm. Preferably the fiber length is between about 20 µm and about 70 µm. The nodular size is preferably not more than about 20 µm. The wall thickness of the tubing depends on the overall diameter of the tubing and is generally selected to be in the range of from about 0.3 mm to about 2 mm. Preferably, the wall thickness is between about 0.3 mm and about 1 mm. It has been observed that tubing having the above-described physical dimensions exhibits a high patency rate without substantial occlusion by fibrin deposition.

For coating the extruded materials of the invention, the perfluorosulfonate copolymer is deposited on at least one surface of the prosthesis. For most tubular prostheses, especially vascular prostheses, the internal surface is most preferably coated to avoid thrombosis or occlusion of the lumen. Depending on the intended application, the external surface may also be coated with the PFS.

The preferred perfluorosulfonate copolymer of the invention is a copolymer of perfluoro-3,6-dioxa-4-methyl-7-octene sulfonyl fluoride and a perfluoromonomer, preferably tetrafluoroethylene. It has been observed that the perfluorosulfonate copolymer should be converted to the form of a sodium salt in order to enable the copolymer to survive the temperatures involved in sintering processes.

The PFS is delivered to the extrudate in liquid form. The PFS liquid for contacting the extrudate includes the PFS to be deposited and a liquid carrier component. The PFS liquid may be a dispersion or a solution of the PFS in the carrier. It is preferred that the PFS be contacted with the extrudate as a solution. The solution may be either colloidal or molecular, most preferably molecular. In other words, for the PFS liquids useful for the invention, the carrier preferably is a solvent capable of dissolving the PFS at a molecular level.

Generally, such carriers must be polar solvents, due to the hydrophilic or polar character of the PFS itself. Accordingly, aqueous solutions are suitable as carriers, as are polar organic compounds that are liquids within the conditions of contact with the material being coated. Aqueous solvents useful according to the invention include water, preferably deionized water, solutions of inorganic salts, and aqueous solvents containing organic compounds having polar qualities, such as alcohols, aldehydes, ketones, organic acids, and mixtures thereof. Polar organic solvents useful according to the invention include polar organic compounds such as alcohols, aldehydes, ketones, organic acids, and mixtures thereof. More preferred polar organic solvents include lower aliphatic alcohols. A highly preferred solvent is a mixture of propanols and a small amount of water, i.e., up to about 10-15% by volume.

Carriers useful according to this embodiment also include certain perhalogenated compounds such as those described in U.S. Patent No. 4,778,723, the disclosure of which is incorporated herein. Preferred perhalogenated compounds include 1,2-dibromotetrafluoroethane and 1,2,2-trichlorotrifluoroethane. Such compounds are not polar but are polarizable, exhibiting polar behavior in the presence of polar compounds and non-polar behavior in the presence of non-polar compounds. Accordingly, such compounds will tend to enhance the dissolution of PFS by dissolving the non-polar fluoropolymeric backbone of the PFS molecules, as well as the polar pendent sulfonic groups.

The PFS may be dissolved or dispersed in the carrier at concentrations ranging from about 0.1 to about 50 weight percent (hereinafter abbreviated wt.%) of PFS in carrier. Preferably the concentration of PFS in the carrier is in the range of from about 0.1 wt% to about 20 wt.%, more preferably from about 0.3 wt.% to about 10 wt.%.

The PFS fluids may be obtained from commercial sources or may be prepared using solid PFS and carriers obtained separately from commercial sources. Conditions suitable for preparing PFS fluids include temperatures ranging from about 10°C to about 50°C, preferably in the range of normal room or ambient temperatures. Pressures suitable for preparing PFS solutions or dispersions range from about atmospheric pressure to about 20 atmospheres, preferably about atmospheric pressure.

Perfluorosulfonate copolymers found to be useful for the invention include a pellet form (mesh 35-80) such as that available as NR-50 from DuPont Co., Wilmington, Delaware; a 5% solution in mixed propanols such as that available from Aldrich Chemical Co., Milwaukee, Wisconsin; and a 10% solution in propanols such as that available from Solution Technology, Mendenhall, Pennsylvania.

The conditions of contacting the fluoropolymer extrudate with the PFS compositions include temperatures ranging from about 10°C to about 110°C. Preferably the temperature of contact will be substantially below the boiling point of the carrier. Pressures suitable for contacting the PFS compositions with the extrudate include pressures in the range of from about atmospheric pressure to about 20 atmospheres, preferably about atmospheric pressure. In cases where a temperature is employed which significantly approaches or exceeds the boiling point of the carrier, a supra-atmospheric pressure may be imposed, limiting carrier loss and permitting contact at reflux conditions.

It has been observed that when a fluoropolymer prosthesis is coated with a liquid containing PFS the temperature of drying of the coated prosthesis can affect the resultant physical properties of the prosthesis. NAFION®-type perfluorosulfonate copolymers are degraded by high temperatures (>250-260°C) and therefore a drying temperature lower than about 250°C is desirable. Nonetheless, an even lower drying temperature has been found to be more desirable for other reasons. It has been found that temperatures between about 50°C and about 110°C are preferable for drying PFS coated prostheses since higher temperatures disturb the integrity of the coating of PFS while temperatures in the preferred range maintain evenness and integrity of the coating on the fluoropolymer prosthesis. In the case where propanols (b.p.∼80°C) are used as the solvent/carrier for the PFS, a drying temperature of about 100°C has proven useful.

A variety of methods may be employed for contacting the extrudate with the PFS compositions. Such methods may be selected from among those known to persons skilled in the art, including such techniques as dipping, immersing, spraying, pouring, or painting. For coating tubular extrudates such as extrudates useful as vascular grafts, either the interior (luminal) or exterior surfaces may be coated, or the PFS may be applied to both the luminal and exterior surfaces.

The coating or film of PFS on the extrudate material may be applied in a single contacting procedure. Alternatively, the coating may be applied in a sequence of contacting procedures, building up the thickness of the coating in a step wise fashion. In using a sequential deposition approach, each application of the PFS composition is followed by a removal of the carrier liquid, allowing the coated material to dry, and optionally sintering the coated material. Furthermore, the coated article may be polished between coatings, to remove imperfections introduced during the coating process.

The carrier liquid is removed from the coated material after the contacting step. Preferably, the carrier liquid is removed immediately after coating, at a temperature of about ambient temperature or higher. Conditions of removal include temperatures ranging from about 10°C to about 110°C depending on the boiling point of the carrier. Pressures suitable for carrier removal include pressures from substantially subatmospheric pressures, e.g., about 20 mm, to about atmospheric pressure.

The prosthesis may be sintered by heating. The sintering may be performed in a continuous fashion with the removal of the carrier liquid or may be performed as a discrete step. The sintering is performed at temperatures in the range of from about 300°C to a temperature above the crystalline melting point of the fluoropolymers in the extrudate. A preferred range of sintering temperatures includes temperatures between about 330°C and about 390°C. Most preferably, the article is sintered at a temperature between about 360°C and about 375°C.

It is known that perfluorosulfonate copolymers such as NAFION® can be thermally degraded at temperatures above about 250-260°C. See Wilkie et al., "Interaction of Poly (Methyl Methacrylate) and Nafions", 42 J. Appl. Polymer Sci. 901-909 (1991). Such degradation involves the release of SO₂ gas and results in the loss of mass in the PFS-containing article. In the case of coated prostheses, significant loss of weight can occur during sintering, producing up to about 10-12% weight loss. Insofar as the PFS is broken down, the advantages of enhanced wettability can be significantly compromised.

Of of the advantages of the invention is that, by converting the PFS copolymer from the acid form to the salt form, this loss of weight is prevented to a large extent. Accordingly, the advantageous physical properties of the PFS-containing article are retained by employing a salt of the PFS copolymer.

While the invention has been described with reference to specific embodiments, it will be apparent to those person having skill in the art that numerous variations, modifications and embodiments are possible, and, accordingly, all such variations, modifications and embodiments are to be regarded as being within the spirit and scope of the present invention.

The aforementioned perfluoroalkoxytetrafluoroethylene polymer is a polymer having a main claim of perfluorinated carbon atoms joined by single bonds: 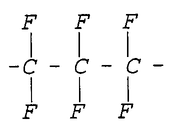
to which main chain are attached a plurality of perfluoroalkoxy moieties: 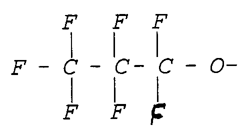
This is possible because tetrafluoroethylene: 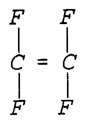
has a double bond which can be copolymerized with a perfluorovinyl ether such as 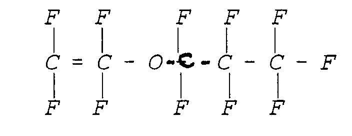
in this case perfluorovinyl (propyl) ether, more properly perfluoro(propyl vinyl ether). Note the similarity to tetrafluoroethylene.

## Claims

1. A process for producing a biocompatible fluoropolymer material, comprising the steps of:
(a) forming an extrusion feedstream comprising (i) a first fluoropolymer component comprising a perfluorosulfonate copolymer, and (ii) a second fluoropolymer component comprising a fluoropolymer; and
(b) extruding said extrusion feedstream to form an extrudate.

2. A process according to Claim 1, wherein the first fluoropolymer component comprises the perfluorosulfonate copolymer and a fluoropolymer.

3. A process according to Claim 1 or 2, wherein the perfluorosulfonate copolymer is a copolymer of perfluoro-3,6-dioxa-4-methyl-7-octene sulfonyl fluoride and a tetrafluoroethylene monomer.

4. A process according to Claim 1, 2 or 3, wherein the second fluoropolymer component comprises a fluoropolymer selected from polytetrafluoroethylene, chlorotrifluoroethylene polymers, fluorinated ethylene-propylene polymers, polyvinylidene fluoride, hexafluoropropylene polymers, perfluoroalkoxytetrafluoroethylene polymers, and mixtures thereof.

5. A process according to any preceding claim, wherein the forming step comprises combining the first and second fluoropolymer components to form a substantially homogeneous extrusion feedstream.

6. A process according to any one of Claims 1 to 4, wherein the forming step comprises co-feeding the first and second fluoropolymer components; and
wherein the extruding step comprises co-extruding the first and second fluoropolymer components without substantially commingling those fluoropolymer components.

7. A process according to any preceding claim, wherein the extruding step comprises extruding the feedstream to form a tubular extrudate.

8. A process according to any preceding claim, which includes the step of stretching the extrudate at a temperature below the crystalline melting point of the extrudate.

9. A process according to any preceding claim, which includes incorporating in the extrusion feedstream a lubricant.

10. A vascular prosthesis comprising a biocompatible fluoropolymer alloy material, the alloy material comprising a blend of (i) a first fluoropolymer including a copolymer of perfluoro-3,6-dioxa-4-methyl-7-octene sulfonyl fluoride and a tetrafluoroethylene monomer, and (ii) a second fluoropolymer, including polytetrafluoroethylene; the alloy material having been extruded to form a tubular extrudate.
